# EUROPEAN PATENT APPLICATION

(11) **EP 4 431 610 A1**
(43) Date of publication of application: **18.09.2024**
(21) Application number: 22892344.7
(22) Date of filing: 05.07.2022
(51) Int. Cl.: C12P 7/64, B01D 29/01, B01D 29/11, B01D 35/02

(54) **METHOD FOR MANUFACTURING FAT/OIL USING YEAST BELONGING TO GENUS LIPOMYCES**

(30) Priority: 12.11.2021 JP 2021184722
(71) Applicant: NISSIN FOODS HOLDINGS CO., LTD., Yodogawa-ku Osaka-shi, Osaka 532-8524 (JP); National University Corporation Kobe University, Kobe-shi, Hyogo 657-8501 (JP)
(72) Inventor: MATSUYAMA, Hideto, Hyogo, 657-8501 (JP); KUMAGAI, Kazuo, Hyogo, 657-8501 (JP); TAKI, Hiroya, Osaka, 532-8524 (JP); MINE, Kentaro, Osaka, 532-8524 (JP)
(74) Representative: Marks & Clerk LLP
(86) International application number: PCT/JP2022/026714
(87) International publication number: WO 2023/084841

(57) **Abstract**

An object of the present invention is to provide a method for producing an alternative to palm oil, the method being adaptable to mass production and requiring low cost. An oil production method of the present invention includes: a step A of culturing a yeast belonging to the genus *Lipomyces*; a step B of disrupting the yeast belonging to the genus *Lipomyces* and obtaining an emulsion; a step C of separating the emulsion into a residue and a liquid by passing the emulsion through a non-woven fabric or a woven fabric; a step D of dissolving the oil contained in the residue into a solvent; and a step E of evaporating the solvent and collecting the oil. The solvent is a solvent mixture of n-hexane and ethanol at a *n-*hexane:ethanol ratio of 2:1 to 5:1. Preferably, the non-woven fabric or the woven fabric has an average pore size of 0.5 to 10 µm and an air permeability of 2 to 45 cm³/(cm²•s).

## Description

### Technical Field

The present invention relates to a method for producing a palm oil analog using a yeast belonging to the genus *Lipomyces.*

### Background Art

Palm oil is a vegetable oil obtained from the fruit of oil palm and is solid at room temperature. Palm oil is characterized in that it contains a large amount of saturated fatty acids and that palmitic acid and oleic acid account for about 80% of total fatty acids. Palm oil is known to be used not only as an edible oil but as a material of margarine, shortening, or soaps. Palm oil is also used as a fried oil for instant fried noodles or snack foods such as potato chips.

Palm oil is the vegetable oil that is produced in the largest quantities in the world. In 2015, 6256 × 10⁴ tons of palm oil were produced in the entire world. Oil palm is a vegetable that grows in tropical areas where the climate is hot and humid. Oil palm is originated in countries of West Africa and Latin America, and at present Malaysia and Indonesia are responsible for 80% or more of palm oil production. Since oil palm bears fruit throughout the year, the yield of oil palm fruit is much greater than that of other vegetable oil sources, and palm oil can be produced as much as 8 to 10 times soybean oil or rapeseed oil per unit cultivated area. Hence, palm oil is inexpensive compared to other vegetable oils and can be supplied stably, for which reason many countries import palm oil.

Oil palm can grow only in equatorial tropical areas where the climate is hot and humid, and regions that can offer growing conditions suitable for oil palm coincide with those where tropical rainforests are distributed. Thus, developing an oil palm plantation inevitably requires cutting down a tropical rainforest, and many tropical rainforests are cut down and lost every year. Massive forest fires have also occurred during development of oil palm plantations. Due to such a loss of tropical rainforests, rare species of wild animals living there are on the verge of extinction.

Additionally, a rapid increase in demand for palm oil has led to workers being placed under poor working conditions or given rise to conflicts over land development between local residents and developers. Thus, palm oil has become a cause of labor problems and human rights problems as well as of global environmental problems.

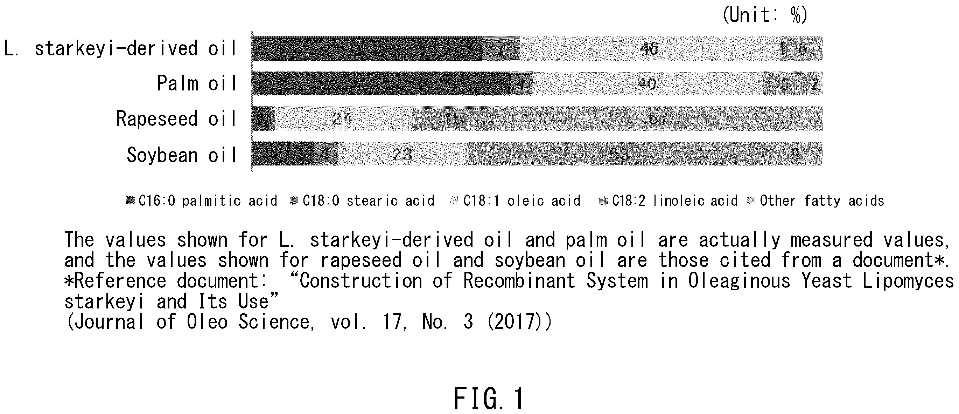

Against such a background, oils alternative to palm oil have been sought in recent years. A yeast belonging to the genus *Lipomyces* is an oil-producing yeast and known to produce an oil which, as shown in FIG. 1, has a fatty acid composition similar to that of palm oil. The oil produced by the yeast belonging to the genus *Lipomyces* is similar also in physical properties to palm oil and is promising as an alternative to palm oil in terms of environment risk, climate change risk, and sustainability.

The oil produced by the yeast belonging to the genus *Lipomyces* is mostly accumulated in the yeast cells. A major technique used for collection of the oil from the yeast cells is to disrupt the dry or wet cells by means such as beads, extract the oil with an organic solvent from the resulting product of the disruption, concentrate the extract and obtain the oil (Non Patent Literature 1).

Known means for separating an emulsified oil into water and an oil is an oil-water separation filter or a non-woven fabric. Patent Literatures 1 and 2 each disclose an oil-water separation filter including a non-woven fabric having: one surface from which a liquid mixture containing water and an oil enters the non-woven fabric; another surface opposite to the one surface; and pores formed between fibers of the non-woven fabric and extending between the two surfaces.

Patent Literature 3 discloses an oil-water separator including a component such as a non-woven fabric or woven fabric which has a hydrophobic surface coated with an oleophilic coating.

### Citation List

### Patent Literature

PTL 1: Japanese Laid-Open Patent Application Publication No. 2019-42707
PTL 2: Japanese Laid-Open Patent Application Publication No. 2020-138195
PTL 3: Japanese Laid-Open Patent Application Publication No. 2012-91168

### Non Patent Literature

NPL 1: Kyle, V. Probst, et al., Evaluation of Green Solvents; Oil Extraction from Oleaginous Yeast Lipomyces starkeyi Using Cyclopentyl Methyl Ether (CPME). Biotechnology Progress. 2017; 33:1096-1103

### Summary of Invention

### Technical Problem

For industrial or practical use of a method for producing an alternative to palm oil (palm oil analog) by means of a yeast belonging to the genus *Lipomyces,* the method needs to be adaptable to mass production. In most of conventional alternative-to-palm oil production methods which have been performed on a laboratory scale, an organic solvent such as chloroform or methanol which cannot be used for edible oil production is used to extract the produced oil from wet cells. Organic solvents that can be used for edible oil production in Japan include *n*-hexane; however, as described below, the rate of oil collection from wet cells through *n*-hexane extraction is low. Thus, when an oil is extracted from cells by means of *n-*hexane, it is necessary to use a technique (dry oil extraction technique) in which the cells are isolated and prepared into dry yeast cells, the dry yeast cells are disrupted, and then the oil is extracted with *n*-hexane.

However, since the yeast belonging to the genus *Lipomyces* contains an oil, the yeast cells cannot be allowed to evenly settle out by means of an industrial-scale centrifuge (up to about 10, 000 × g) and are difficult to collect. To construct a specialized high-speed centrifuge capable of processing a huge volume of yeast-containing liquid and allowing the yeast cells to evenly settle out entails a lot of equipment investment and high running cost and is not economically practical.

When drying yeast cells to prepare dry yeast cells, it is preferable to employ vacuum-freeze drying. However, the use of a large-scale vacuum-freeze dryer entails a lot of equipment investment and high running cost. Additionally, such a vacuum-freeze dryer is a batch-type machine and thus has limited throughput. As for the subsequent disruption of the dry yeast cells, many of dry process disruptors such as ball mills are of the batch type and have the drawback of requiring solvent-resistant design. Even with the use of a continuous-type disruptor such as a jet mill, the disruptor is often clogged with an oil which is highly viscous, and this makes it difficult to implement a continuous process. Thus, the dry oil extraction technique is not suitable for adapting an alternative-to-palm oil production method to mass production.

When yeast cells are disrupted in a yeast culture fluid without collecting the yeast cells from the fluid, an oil produced in the yeast cells leaks out of the yeast cells and induces emulsification. This emulsification is considered to occur due to the influence of a large amount of water and the influence of proteins or polysaccharides derived from the yeast. Separation and collection of the oil form the emulsion are difficult to accomplish even by a wet oil extraction technique using an organic solvent or a demulsifier, and the rate of oil collection is notably low. That is, collection of an alternative to palm oil from an emulsion resulting from disruption of wet yeast cells is impossible to achieve at a practical level.

As described above, any conventional alternative-to-palm oil production methods using a yeast belonging to the genus *Lipomyces* are difficult to adapt to mass production of edible oils. There is a demand for development of a method for producing an alternative to palm oil, the method being adaptable to mass production and requiring low cost.

### Solution to Problem

The present inventors have conducted intensive studies to solve the problems as described above. As a result, the present inventors have found that practical collection of an alternative to palm oil from an emulsion, which is almost impossible by conventional techniques, can be accomplished by culturing a yeast belonging to the genus *Lipomyces,* disrupting the cultured yeast cells in a liquid to give an emulsion, filtering the emulsion through a non-woven fabric or woven fabric, and collecting an oil from the residue on the non-woven fabric or woven fabric by means of a particular organic solvent (an organic solvent mixture prepared by mixing two organic solvents at a particular volume ratio). Based on this finding, the inventors have completed the present invention.

Specifically, the present invention relates to an oil production method including:
a step A of culturing a yeast belonging to the genus *Lipomyces* and allowing the yeast to produce an oil;
a step B of disrupting the yeast belonging to the genus *Lipomyces* and obtaining an emulsion;
a step C of separating the emulsion into a residue and a liquid by passing the emulsion through a non-woven fabric or a woven fabric;
a step D of distributing the oil contained in the residue into n-hexane and ethanol and collecting a n-hexane layer; and
a step E of evaporating the n-hexane and collecting the oil, wherein
a volume ratio of the n-hexane to the ethanol is from 2:1 to 5:1.

Collection of an alternative to palm oil from an emulsion resulting from disrupting a yeast belonging to the genus *Lipomyces* in a liquid was impossible to achieve at a practical collection rate with the use of a solvent extraction technique using an organic solvent or a separation technique using a demulsifier. However, the yeast cell residue can be separated from the liquid by passing the emulsion through a non-woven fabric or woven fabric, preferably a non-woven fabric or woven fabric having a given average pore size and a given air permeability (air permeability measured based on Method A (Frazier method) of air permeability test as specified in JIS L 1096). With the use of a particular organic solvent (solvent mixture of *n-*hexane and ethanol), the alternative to palm oil produced by the yeast belonging to the genus *Lipomyces* is dissolved in n-hexane, and this enables efficient collection of the alternative to palm oil from the emulsion.

The non-woven fabric or the woven fabric preferably has an average pore size of 0.5 to 10 µm and an air permeability of 2 to 45 cm³/(cm²•s).

The yeast belonging to the genus *Lipomyces* may be any yeast that produces the oil and is particularly preferably *Lipomyces starkeyi.*

In the step C, the emulsion is preferably passed through the non-woven fabric or the woven fabric at a pressure of 5 to 200 kPa, preferably 10 to 100 kPa.

### Advantageous Effects of Invention

With the use of the oil production method of the present invention, an alternative to palm oil, which can be used, for example, as an edible oil or a material of cosmetics, can be stably produced at low cost and in large quantities.

### Brief Description of Drawings

FIG. 1 is a graph showing comparison of the fatty acid composition of an oil (alternative to palm oil) produced by *Lipomyces starkeyi* with those of palm oil, rapeseed oil, and soybean oil.

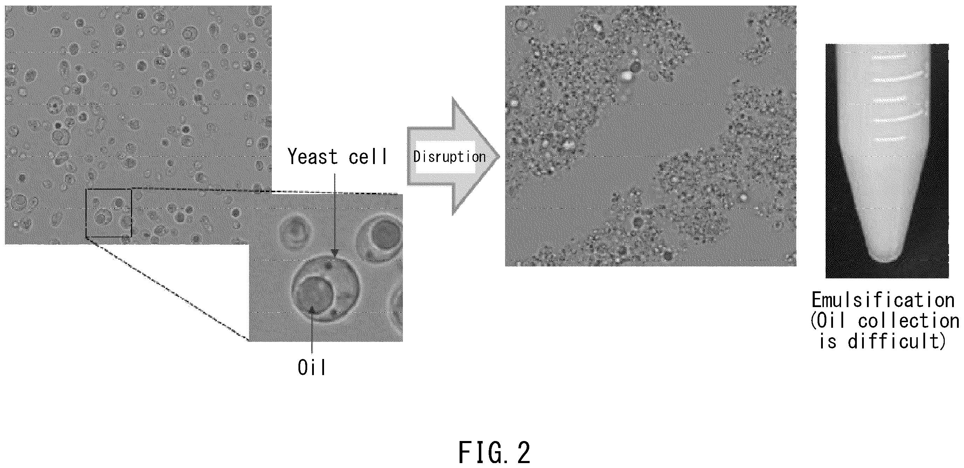

FIG. 2 shows a microscopic image of *Lipomyces starkeyi,* a microscopic image taken after disruption of yeast cells by a homogenizer, and a photograph of the appearance of an emulsion resulting from the disruption.

### Description of Embodiments

Embodiments of the present invention will be described with reference to the drawings.

An oil production method of the present invention includes:
a step A of culturing a yeast belonging to the genus *Lipomyces* and allowing the yeast to produce an oil;
a step B of disrupting the yeast belonging to the genus *Lipomyces* and obtaining an emulsion;
a step C of separating the emulsion into a residue and a liquid by passing the emulsion through a non-woven fabric or a woven fabric;
a step D of distributing the oil contained in the residue into *n*-hexane and ethanol and collecting a *n*-hexane layer; and
a step E of evaporating the *n*-hexane and collecting the oil.

### <Step A>

A yeast belonging to the genus *Lipomyces* is cultured and allowed to produce an alternative to palm oil (palm oil analog). As shown in FIG. 1, the alternative to palm oil produced by the yeast belonging to the genus *Lipomyces* has a fatty acid composition similar to that of palm oil. The fatty acid composition of the alternative to palm oil is little dependent on culture conditions. The yeast belonging to the genus *Lipomyces* can be cultured using a known culture method suitable for yeasts. The culture is preferably carried out at a temperature of 25 to 30°C and an aeration rate per liter of culture fluid of 1 to 3 L/min under stirring at 100 to 600 rpm. In the step A, the culture is preferably continued until the number of the cells of the yeast belonging to the genus *Lipomyces* reached 4.0 × 10⁸ to 4.0 × 10⁹/mL.

### <Step B>

After the step A, the yeast belonging to the genus *Lipomyces* is disrupted in a liquid such as a culture fluid to prepare an emulsion. The yeast belonging to the genus *Lipomyces* may be disrupted in the culture fluid used in the step A. Alternatively, the yeast belonging to the genus *Lipomyces* may be collected from the culture fluid, transferred to another liquid, and then disrupted in the other liquid. The yeast cells can be disrupted using a known disruption device such as a jet mill disruptor or an ultrasonic homogenizer. Two or more disruption means may be used in combination, and an enzyme treatment and a disruption device may be used in combination. In the step B, the disruption of the yeast cells is preferably continued until yeast cells retaining their shape become unobservable by microscopic inspection in several fields of view.

### <Step C>

The emulsion prepared in the step B is passed through a non-woven fabric or a woven fabric to separate the emulsion into a residue including the yeast cells and a water-soluble liquid. The non-woven fabric or woven fabric used in the step C preferably has an average pore size of 0.5 to 10 µm and an air permeability (air permeability measured based on Method A (Frazier method) of air permeability test as specified in JIS L 1096) of 2 to 45 cm³/(cm²•s). If the air permeability is less than 2 cm³/(cm²•s), the passage speed of the emulsion decreases considerably, and the water-soluble component to be removed has difficulty passing through the non-woven fabric or woven fabric. If the air permeability is more than 45 cm³/(cm²•s), the emulsion passes through the non-woven fabric or woven fabric too smoothly and the cell residue is difficult to catch. The non-woven fabric or woven fabric may be shaped as a flat sheet or a tube. The non-woven fabric or woven fabric need not be coated with a special coating such as a hydrophobic membrane and may be a commercially-available product made of a material such as polypropylene or polyester. In terms of cost, the non-woven fabric is more preferred than the woven fabric when the fabrics are similar in performance.

The emulsion is preferably passed through the non-woven fabric or woven fabric at a pressure of 5 to 200 kPa, more preferably 10 to 100 kPa. If the pressure is less than 5 kPa, the passage of the emulsion requires a lot of time. If the pressure is more than 200 kPa, the non-woven fabric or woven fabric is likely to be physically damaged so that the resulting interstice permits passage of a substance that should be blocked from passing through the non-woven fabric or woven fabric.

As a result of passing the emulsion through the non-woven fabric or woven fabric, the yeast cell residue is caught on the non-woven fabric or woven fabric, and the water and water-soluble component pass through the non-woven fabric or woven fabric. The alternative to palm oil produced by the yeast belonging to the genus *Lipomyces* is caught together with the yeast cell residue on the non-woven fabric or woven fabric. If the yeast cell-containing liquid is passed through the non-woven fabric or woven fabric directly (without disruption of the cells), the non-woven fabric or woven fabric readily becomes clogged since the cell size distribution is wide. In the case where the emulsion is passed through the non-woven fabric or woven fabric, the frequency of occurrence of clogging of the non-woven fabric or woven fabric is reduced because the disruption has narrowed the cell size distribution. The first technical feature of the alternative-to-palm oil production method of the present invention is that in the step C, the alternative to palm oil is caught together with the cell residue on the non-woven fabric or woven fabric by passing the emulsion through the non-woven fabric or woven fabric.

### <Step D>

The residue caught on the non-woven fabric or woven fabric in the step C is mixed with a solvent mixture of *n*-hexane and ethanol at a *n*-hexane:ethanol ratio of 2:1 to 5:1 (volume ratio). In this mixing process, the alternative to palm oil contained in the residue is dissolved in a n-hexane layer of the two solvent layers. To increase the rate of collection of the alternative to palm oil, the residue and the solvent mixture are preferably subjected to a mixing operation such as stirring to disperse the residue well in the solvent mixture. The solvent mixture mixed with the residue is allowed to stand, in consequence of which the mixture is divided into a *n-*hexane layer (upper layer) and a layer of a mixture of ethanol and water (lower layer). By collecting the upper layer, only *n*-hexane in which the alternative to palm oil is dissolved can be collected.

When pressing the fruit of oil palm and extracting palm oil, it is common practice to use n-hexane as an extraction solvent. The second technical feature of the alternative-to-palm oil production method of the present invention is that in the step D, a solvent mixture of *n-*hexane and ethanol at a *n*-hexane:ethanol ratio of 2:1 to 5:1 (volume ratio) is used as an extraction solvent instead of *n*-hexane alone. By virtue of the fact that a solvent mixture containing *n*-hexane which is a non-hydrophilic solvent and ethanol which is a hydrophilic solvent in given proportions is used as an extraction solvent in the step D, the rate of collection of the alternative to palm oil can be significantly higher than when *n*-hexane is used alone.

### <Step E>

The alternative to palm oil is collected by evaporating *n*-hexane from the upper layer liquid collected in the step D. The method used to evaporate *n*-hexane may be a known method such as distillation under reduced pressure.

The collected alternative to palm oil may, if necessary, be subjected to a known purification treatment such as deacidification, degumming, or deodorization. The used solvents may, if necessary, be recovered and reused.

### First Embodiment: Embodiment in Which Step B Includes Disrupting Yeast in Purified Water

### [Reference Example]

### <Step A>

A volume of 1.5 L of culture medium containing 5 w/v% yeast extract as a nitrogen source and 10 w/v% D(+)-glucose as a carbon source was prepared, and the pH of the culture medium was adjusted to 6.0 using a 6 N aqueous solution of sodium hydroxide. *Lipomyces starkeyi* was added to the culture medium with the adjusted pH and aerobically cultured using a microbial culture device (ABLE Corporation, BMS-C (culture vessel with a volume of 5 L)) at 30°C for 7 days (stirring speed: 600 rpm, aeration rate: 3 L/min). The D(+)-glucose concentration in the culture medium was measured four times every day, and D(+)-glucose was added to maintain the D(+)-glucose concentration at 10 w/v%.

### <Step B>

After the culture, the culture fluid was centrifuged (10,000 × g, 60 minutes) to collect wet cells. Suspended cells that did not evenly settle out were collected by scooping them with a medicine spoon. The collected wet cells were subjected to vacuum-freeze drying to obtain dry cells. The dry cells were dispersed in purified water in a tube to give a dry cell content of 10 w/v%, and thus 15 mL of *Lipomyces starkeyi-containing* liquid (simulated culture fluid) was prepared. This liquid was prepared taking into account a *Lipomyces starkeyi* cell concentration serving as a reference based on which the culture in the step A is ended. To this liquid was added 0.5% (w/v) of glucanase (TUNICASE SD-FN, manufactured by Amano Enzyme Inc.), and the liquid was enzyme-treated at 50°C overnight. After that, the liquid was subjected to a wet disruption process using an ultrasonic homogenizer (QSONICA Q500 manufactured by WakenBtech Co., Ltd.) to disrupt the cells of *Lipomyces starkeyi* (40% output, 60 minutes). As a result of being stirred, the liquid turned into a homogeneous yellowish-white emulsion.

A theoretical value was determined for calculation of the rate of oil collection from the emulsion. There is no way to collect and quantify 100% of the oil contained in the emulsion; thus, the theoretical value was determined based on the amount of triglycerides which stably exist regardless of whether emulsification takes place. Specifically, the rate of oil collection was calculated from a result of gas chromatography-mass spectrometry of triglycerides. The triglyceride content in 1 mL of the emulsion was found to be 1.9% (w/v). An oil obtained by disruption of dry yeast cells with the use of Multi-Beads Shocker (Yasui Kikai Corporation) and the subsequent *n*-hexane extraction was also assayed as a sample in the manner as described above, and the triglyceride content in the oil was found to be 82.7% (w/v) (it can be envisaged that diglycerides, monoglycerides, and free fatty acids were also contained). Based on the above results, the weight of the oil (theoretical value) obtained from 15 mL of the emulsion is calculated as 15 × 0.019/0.827 = 0.35 (g).

### <Step C>

A non-woven fabric was mounted to a stirred cell having a filtration region with a diameter of 47 mm, and in the stirred cell 15 mL of the emulsion was subjected to pressure filtration (pressure = 30 kPa) using nitrogen gas. The non-woven fabric used was SYNTEX^{™} nano 6 manufactured by Mitsui Chemicals, Inc. (average pore size = 5.6 µm, Frazier air permeability = 10 cm³/(cm²•s), a melt-blown non-woven fabric made of polypropylene). The cell residue was caught on the non-woven fabric, while the liquid passed through the non-woven fabric.

### <Step D>

The cell residue caught on the non-woven fabric was gathered by means of a medicine spoon and collected into a conical tube. A volume of 10 mL of a solvent mixture of *n*-hexane and ethanol at a *n*-hexane:ethanol ratio of 1:1 (volume ratio) was added into the conical tube, the contents of which were stirred for 1 minute and then allowed to stand for 15 minutes. After that, the upper layer (*n*-hexane layer) was collected by means of a pipette.

### <Step E>

The collected upper layer was put into a conical tube and heated using Dry ThermoUnit (DTU-1CN, manufactured by Taitec Corporation) at 90°C for 60 minutes to evaporate the solvent. The weight of the alternative to palm oil remaining in the container was measured, and the rate of oil collection (%) was calculated as (measured value/theoretical value) × 100.

### [Example 1]

All procedures were performed in the same manner as in Reference Example, except that the solvent mixture used in the step D was 10 mL of a solvent mixture of *n*-hexane and ethanol at a *n*-hexane:ethanol ratio of 2:1 (volume ratio).

### [Example 2]

All procedures were performed in the same manner as in Reference Example, except that the solvent mixture used in the step D was 10 mL of a solvent mixture of *n*-hexane and ethanol at a *n*-hexane:ethanol ratio of 3:1 (volume ratio).

### [Example 3]

All procedures were performed in the same manner as in Reference Example, except that the solvent mixture used in the step D was 10 mL of a solvent mixture of *n*-hexane and ethanol at a *n*-hexane:ethanol ratio of 5:1 (volume ratio).

### [Comparative Example 1]

All procedures were performed in the same manner as in Reference Example, except that the solvent used in the step D was 10 mL of *n*-hexane.

### [Comparative Example 2]

All procedures were performed in the same manner as in Reference Example, except that the solvent used in the step D was 10 mL of ethanol.

### [Comparative Example 3]

All procedures were performed in the same manner as in Reference Example, except that the solvent used in the step D was 10 mL of isopropanol.

### [Comparative Example 4]

All procedures were performed in the same manner as in Reference Example, except that the solvent mixture used in the step D was 10 mL of a solvent mixture of *n*-hexane and ethanol at a *n*-hexane:ethanol ratio of 10:1 (volume ratio).

### [Comparative Example 5]

All procedures were performed in the same manner as in Reference Example, except that the solvent mixture used in the step D was 10 mL of a solvent mixture of *n*-hexane and isopropanol at a *n*-hexane:isopropanol ratio of 1:1 (volume ratio).

### [Comparative Example 6]

All procedures were performed in the same manner as in Reference Example, except that the solvent mixture used in the step D was 10 mL of a solvent mixture of *n*-hexane and isopropanol at a *n*-hexane:isopropanol ratio of 2:1 (volume ratio).

### [Comparative Example 7]

All procedures were performed in the same manner as in Reference Example, except that the solvent mixture used in the step D was 10 mL of a solvent mixture of *n*-hexane and isopropanol at a *n*-hexane:isopropanol ratio of 3:1 (volume ratio).

### [Comparative Example 8]

All procedures were performed in the same manner as in Reference Example, except that the solvent mixture used in the step D was 10 mL of a solvent mixture of *n*-hexane and isopropanol at a *n*-hexane:isopropanol ratio of 5:1 (volume ratio).

### [Comparative Example 9]

All procedures were performed in the same manner as in Reference Example, except that the solvent mixture used in the step D was 10 mL of a solvent mixture of isopropanol and ethanol at an isopropanol:ethanol ratio of 1:1 (volume ratio).

### [Comparative Example 10]

All procedures were performed in the same manner as in Reference Example, except that the solvent mixture used in the step D was 10 mL of a solvent mixture of isopropanol and ethanol at an isopropanol:ethanol ratio of 2:1 (volume ratio).

### [Comparative Example 11]

All procedures were performed in the same manner as in Reference Example, except that the solvent mixture used in the step D was 10 mL of a solvent mixture of isopropanol and ethanol at an isopropanol:ethanol ratio of 3:1 (volume ratio).

### [Comparative Example 12]

All procedures were performed in the same manner as in Reference Example, except that the solvent mixture used in the step D was 10 mL of a solvent mixture of isopropanol and ethanol at an isopropanol:ethanol ratio of 5:1 (volume ratio).

### <Comparative Examples Where Steps C and D Were Not Performed>

### [Comparative Example 13]

After the step B in Reference Example, the step C was not performed, but instead 10 mL of n-hexane was added as a solvent to the emulsion, which was then stirred for 1 minute. The emulsion was then allowed to stand for 15 minutes, after which collection of the solvent layer (upper layer) by means of a pipette was performed in place of the step D. The collected solvent layer was subjected to procedures which were the same as those of the step E in Reference Example.

### [Comparative Example 14]

Procedures were performed in the same manner as in Comparative Example 13, except that the solvent added to the emulsion was 10 mL of ethanol.

### [Comparative Example 15]

Procedures were performed in the same manner as in Comparative Example 13, except that the solvent added to the emulsion was 10 mL of isopropanol.

### [Comparative Example 16]

Procedures were performed in the same manner as in Comparative Example 13, except that the solvent added to the emulsion was 10 mL of a solvent mixture of *n*-hexane and ethanol at a *n*-hexane:ethanol ratio of 1:1 (volume ratio).

### [Comparative Example 17]

Procedures were performed in the same manner as in Comparative Example 13, except that the solvent added to the emulsion was 10 mL of a solvent mixture of *n*-hexane and ethanol at a *n*-hexane:ethanol ratio of 2:1 (volume ratio).

### [Comparative Example 18]

Procedures were performed in the same manner as in Comparative Example 13, except that the solvent added to the emulsion was 10 mL of a solvent mixture of *n*-hexane and ethanol at a *n*-hexane:ethanol ratio of 3:1 (volume ratio).

### [Comparative Example 19]

Procedures were performed in the same manner as in Comparative Example 13, except that the solvent added to the emulsion was 10 mL of a solvent mixture of *n*-hexane and ethanol at a *n*-hexane:ethanol ratio of 5:1 (volume ratio).

### [Comparative Example 20]

Procedures were performed in the same manner as in Comparative Example 13, except that the solvent added to the emulsion was 10 mL of a solvent mixture of *n*-hexane and ethanol at a *n*-hexane:ethanol ratio of 10:1 (volume ratio).

### [Comparative Example 21]

Procedures were performed in the same manner as in Comparative Example 13, except that the solvent added to the emulsion was 10 mL of a solvent mixture of *n*-hexane and isopropanol at a *n*-hexane:isopropanol ratio of 1:1 (volume ratio).

### [Comparative Example 22]

Procedures were performed in the same manner as in Comparative Example 13, except that the solvent added to the emulsion was 10 mL of a solvent mixture of *n*-hexane and isopropanol at a *n*-hexane:isopropanol ratio of 2:1 (volume ratio).

### [Comparative Example 23]

Procedures were performed in the same manner as in Comparative Example 13, except that the solvent added to the emulsion was 10 mL of a solvent mixture of *n*-hexane and isopropanol at a *n*-hexane:isopropanol ratio of 3:1 (volume ratio).

### [Comparative Example 24]

Procedures were performed in the same manner as in Comparative Example 13, except that the solvent added to the emulsion was 10 mL of a solvent mixture of *n*-hexane and isopropanol at a *n*-hexane:isopropanol ratio of 5:1 (volume ratio).

### [Comparative Example 25]

Procedures were performed in the same manner as in Comparative Example 13, except that the solvent added to the emulsion was 10 mL of a solvent mixture of isopropanol and ethanol at an isopropanol:ethanol ratio of 1:1 (volume ratio).

### [Comparative Example 26]

Procedures were performed in the same manner as in Comparative Example 13, except that the solvent added to the emulsion was 10 mL of a solvent mixture of isopropanol and ethanol at an isopropanol:ethanol ratio of 2:1 (volume ratio).

### [Comparative Example 27]

Procedures were performed in the same manner as in Comparative Example 13, except that the solvent added to the emulsion was 10 mL of a solvent mixture of isopropanol and ethanol at an isopropanol:ethanol ratio of 3:1 (volume ratio).

### [Comparative Example 28]

Procedures were performed in the same manner as in Comparative Example 13, except that the solvent added to the emulsion was 10 mL of a solvent mixture of isopropanol and ethanol at an isopropanol:ethanol ratio of 5:1 (volume ratio).

Table 1 lists the values of the rate of oil collection (%) in Reference Example, Examples 1 to 3, and Comparative Examples 1 to 28. In Reference Example and Examples 1 to 3, the rate of oil collection was 81.2% or more; in particular, in Examples 1 and 2, the rate of oil collection was very high and 98% or more. It has been confirmed that the rate of oil collection is 80% or more when the *n*-hexane:ethanol ratio is in the range of 1:1 to 5:1 and 90% or more when the *n*-hexane:ethanol ratio is in the range of 1.5:1 to 4:1.

In Comparative Examples 1 to 12, which were different from Reference Example and Examples 1 to 3 only in the solvent used in the step D, the rate of oil collection (%) was at most 45.6%. Thus, it has been confirmed that the rate of oil collection significantly depends on the type of the solvent used in the step D.

**[Table 1]**

| Solvent used | With steps C and D | Rate of oil collection (%) | Without steps C and D | Rate of oil collection (%) |
|---|---|---|---|---|
| *n* -Hexane | Comparative Example 1 | 43.5 | Comparative Example 13 | 1.5 |
| Ethanol | Comparative Example 2 | 0 | Comparative Example 14 | 0 |
| Isopropanol | Comparative Example 3 | 40.6 | Comparative Example 15 | 0 |
| *n* -Hexane:Ethanol= 1:1 | Reference Example | **81.2** | Comparative Example 16 | 1.9 |
| *n* -Hexane:Ethanol= 2:1 | Example 1 | **98.7** | Comparative Example 17 | 0 |
| *n* -Hexane:Ethanol= 3:1 | Example 2 | **98.6** | Comparative Example 18 | 1.9 |
| *n* -Hexane:Ethanol= 5:1 | Example 3 | **87.0** | Comparative Example 19 | 0 |
| *n* -Hexane:Ethanol= 10:1 | Comparative Example 4 | 37.5 | Comparative Example 20 | 0 |
| *n* -Hexane:Isopropanol= 1:1 | Comparative Example 5 | 45.6 | Comparative Example 21 | 13.3 |
| *n* -Hexane:Isopropanol = 2:1 | Comparative Example 6 | 13.3 | Comparative Example 22 | 17.1 |
| *n* -Hexane:Isopropanol = 3:1 | Comparative Example 7 | 34.2 | Comparative Example 23 | 13.3 |
| *n* -Hexane:Isopropanol = 5:1 | Comparative Example 8 | 41.8 | Comparative Example 24 | 0 |
| IsopropanolEthanol = 1:1 | Comparative Example 9 | 10.0 | Comparative Example 25 | 0 |
| IsopropanolEthanol = 2:1 | Comparative Example 10 | 10.0 | Comparative Example 26 | 0 |
| IsopropanolEthanol = 3:1 | Comparative Example 11 | 17.5 | Comparative Example 27 | 0 |
| IsopropanolEthanol = 5:1 | Comparative Example 12 | 12.5 | Comparative Example 28 | 0 |

In Comparative Examples 13 to 28, where the steps C an D were performed but instead collection of the alternative to palm oil from the emulsion obtained in the step B was attempted using a solvent, the rate of oil collection was at most 17.1%. In particular, in Comparative Examples 16 to 19, where the solvent mixtures used were the same as those of Examples 1 to 4, the rate of oil collection was as low as 0 to 1.9%.

As previously stated, it is common practice to use n-hexane as an extraction solvent when pressing the fruit of oil palm and extracting palm oil. However, in the case where *n-*hexane was used to collect the alternative to palm oil from the emulsion obtained in the step B (Comparative Example 13), the rate of oil collection was 1.5%. In the case where n-hexane was used as the solvent in the step D (Comparative Example 1), the rate of oil collection was 43.5%; this value was about half of the values of the rate of oil collection in Reference Example and Examples 1 to 3.

### (Influence of Type of Non-woven Fabric)

In Reference Example and Examples 1 to 3, the non-woven fabric used in the step C was a melt-blown non-woven fabric made of polypropylene and having an average pore size of 5.6 µm and a Frazier air permeability of 10 cm³/(cm²•s). When a melt-blown non-woven fabric made of polypropylene and having an average pore size of 0.5 to 10 µm and a Frazier air permeability of 2 to 45 cm³/(cm²•s) was used (examples of such a melt-blown non-woven fabric include SYNTEX^{™} nano 2 (average pore size = 2.2 µm, Frazier air permeability = 7.1 cm³/(cm²•s)) and nano 9 (average pore size = 7.0 µm, Frazier air permeability = 12 cm³/(cm²•s)) which are manufactured by Mitsui Chemicals, Inc.), the rate of oil collection was similar to that in Reference Example and in Examples 1 to 3. When a non-woven fabric that does not meet the above requirements was used, the passage speed was significantly slow or the oil-containing cell residue passed through the non-woven fabric, and the rate of collection was 0%.

### (Use of Demulsifier)

Demulsifiers for foods (RYOTO Sugar Ester P-1670 (sucrose fatty acid ester) manufactured by Mitsubishi Chemical Corporation, and SY-Glyster MO-3S, SY-Glyster MO-5S, and SY-Glyster MO-7S (all of which are polyglyceryl oleate) manufactured by Sakamoto Yakuhin Kogyo Co., Ltd.) were individually added to the emulsion obtained in the step B in an amount of 0.1 to 1.0 w/v%. After the addition of the demulsifier, the emulsion was heated or centrifuged (6,000 × g); however, the liquid phase was not separated, and it was impossible to collect the alternative to palm oil by obtaining only a solvent layer.

### (Disruption Process Using Jet Mill Disruptor)

In Reference Examples 1 to 3, in the step B, a wet disruption process was performed in which the *Lipomyces starkeyi-containing* liquid was stirred by an ultrasonic homogenizer to disrupt the *Lipomyces starkeyi* cells. In another experiment example where the *Lipomyces starkeyi*-containing liquid was subjected to a disruption process using a jet mill disruptor until complete disruption of the *Lipomyces starkeyi* cells was observed, the rate of oil collection (%) was similar to that in Reference Example and Examples 1 to 3.

### Second Embodiment: Embodiment in Which Step B Includes Disrupting Yeast in Culture Fluid

### [Example 4]

### <Step A>

*Lipomyces starkeyi* was cultured in the same manner as in Reference Example until the number of the *Lipomyces starkeyi* cells reached 4.0 × 10⁸ to 4.0 × 10⁹/mL.

### <Step B>

After the culture, the *Lipomyces starkeyi* cells in the culture fluid were subjected to a wet disruption process (output 200 MPa, 10 pass) using a high-pressure homogenizer (NAGS 20 manufactured by Jokoh Co., Ltd.). As a result of the wet disruption process, the liquid turned into a homogeneous yellowish-white emulsion. The wet disruption process may, as in Reference Example, be preceded by an enzyme treatment in which 0.5% (w/v) of glucanase (TUNICASE SD-FN, manufactured by Amano Enzyme Inc.) is added to the culture fluid and the fluid is enzyme-treated at 50°C overnight. This enzyme treatment can reduce the time required for the wet disruption process using the high-pressure homogenizer.

### <Steps C to E>

All the subsequent procedures were performed in the same manner as in Reference Example, except that the solvent mixture used in the step D was 10 mL of a solvent mixture of *n-*hexane and ethanol at a n-hexane:ethanol ratio of 2:1 (volume ratio). The alternative to palm oil was collected in this way, and the rate of oil collection was calculated.

### [Example 5]

All procedures were performed in the same manner as in Example 4, except that the solvent mixture used in the step D was 10 mL of a solvent mixture of n-hexane and ethanol at a *n*-hexane:ethanol ratio of 3:1 (volume ratio).

### [Example 6]

All procedures were performed in the same manner as in Example 4, except that the solvent mixture used in the step D was 10 mL of a solvent mixture of n-hexane and ethanol at a *n*-hexane:ethanol ratio of 5:1 (volume ratio).

### [Comparative Example 29]

All procedures were performed in the same manner as in Example 4, except that the solvent used in the step D was 10 mL of n-hexane.

### [Comparative Example 30]

All procedures were performed in the same manner as in Example 4, except that the solvent used in the step D was 10 mL of ethanol.

### [Comparative Example 31]

All procedures were performed in the same manner as in Example 4, except that the solvent used in the step D was 10 mL of isopropanol.

### [Comparative Example 32]

All procedures were performed in the same manner as in Example 4, except that the solvent mixture used in the step D was 10 mL of a solvent mixture of *n*-hexane and ethanol at a *n*-hexane:ethanol ratio of 1:1 (volume ratio).

### [Comparative Example 33]

All procedures were performed in the same manner as in Example 4, except that the solvent mixture used in the step D was 10 mL of a solvent mixture of *n*-hexane and ethanol at a *n*-hexane:ethanol ratio of 6:1 (volume ratio).

### [Comparative Example 34]

All procedures were performed in the same manner as in Example 4, except that the solvent mixture used in the step D was 10 mL of a solvent mixture of *n*-hexane and ethanol at a *n*-hexane:ethanol ratio of 10:1 (volume ratio).

### [Comparative Example 35]

All procedures were performed in the same manner as in Example 4, except that the solvent mixture used in the step D was 10 mL of a solvent mixture of *n*-hexane and isopropanol at a *n*-hexane:isopropanol ratio of 1:1 (volume ratio).

### [Comparative Example 36]

All procedures were performed in the same manner as in Example 4, except that the solvent mixture used in the step D was 10 mL of a solvent mixture of *n*-hexane and isopropanol at a *n*-hexane:isopropanol ratio of 3:1 (volume ratio).

### [Comparative Example 37]

All procedures were performed in the same manner as in Example 4, except that the solvent mixture used in the step D was 10 mL of a solvent mixture of *n*-hexane and isopropanol at a *n*-hexane:isopropanol ratio of 10:1 (volume ratio).

### [Comparative Example 38]

All procedures were performed in the same manner as in Example 4, except that the solvent mixture used in the step D was 10 mL of a solvent mixture of isopropanol and ethanol at an isopropanol: ethanol ratio of 1:1 (volume ratio).

### [Comparative Example 39]

All procedures were performed in the same manner as in Example 4, except that the solvent mixture used in the step D was 10 mL of a solvent mixture of isopropanol and ethanol at an isopropanol: ethanol ratio of 3:1 (volume ratio).

### [Comparative Example 40]

All procedures were performed in the same manner as in Example 4, except that the solvent mixture used in the step D was 10 mL of a solvent mixture of isopropanol and ethanol at an isopropanol:ethanol ratio of 10:1 (volume ratio).

### <Comparative Examples Where Steps C and D Were Not Performed>

### [Comparative Example 41]

After the step B in Reference Example, the step C was not performed, but instead 10 mL of n-hexane was added as a solvent to the emulsion, which was then stirred for 1 minute. The emulsion was then allowed to stand for 15 minutes, after which collection of the solvent layer (upper layer) by means of a pipette was performed in place of the step D. The collected solvent layer was subjected to procedures which were the same as those of the step E in Reference Example.

### [Comparative Example 42]

Procedures were performed in the same manner as in Comparative Example 41, except that the solvent added to the emulsion was 10 mL of ethanol.

### [Comparative Example 43]

Procedures were performed in the same manner as in Comparative Example 41, except that the solvent added to the emulsion was 10 mL of isopropanol.

### [Comparative Example 44]

Procedures were performed in the same manner as in Comparative Example 41, except that the solvent added to the emulsion was 10 mL of a solvent mixture of *n*-hexane and ethanol at a *n*-hexane:ethanol ratio of 1:1 (volume ratio).

### [Comparative Example 45]

Procedures were performed in the same manner as in Comparative Example 41, except that the solvent added to the emulsion was 10 mL of a solvent mixture of *n*-hexane and ethanol at a *n*-hexane:ethanol ratio of 2:1 (volume ratio).

### [Comparative Example 46]

Procedures were performed in the same manner as in Comparative Example 41, except that the solvent added to the emulsion was 10 mL of a solvent mixture of *n*-hexane and ethanol at a *n*-hexane:ethanol ratio of 3:1 (volume ratio).

### [Comparative Example 47]

Procedures were performed in the same manner as in Comparative Example 41, except that the solvent added to the emulsion was 10 mL of a solvent mixture of *n*-hexane and ethanol at a *n*-hexane:ethanol ratio of 5:1 (volume ratio).

### [Comparative Example 48]

Procedures were performed in the same manner as in Comparative Example 41, except that the solvent added to the emulsion was 10 mL of a solvent mixture of *n*-hexane and ethanol at a *n*-hexane:ethanol ratio of 6:1 (volume ratio).

### [Comparative Example 49]

Procedures were performed in the same manner as in Comparative Example 41, except that the solvent added to the emulsion was 10 mL of a solvent mixture of *n*-hexane and ethanol at a *n*-hexane:ethanol ratio of 10:1 (volume ratio).

### [Comparative Example 50]

Procedures were performed in the same manner as in Comparative Example 41, except that the solvent added to the emulsion was 10 mL of a solvent mixture of *n*-hexane and isopropanol at a *n*-hexane:isopropanol ratio of 1:1 (volume ratio).

### [Comparative Example 51]

Procedures were performed in the same manner as in Comparative Example 41, except that the solvent added to the emulsion was 10 mL of a solvent mixture of *n*-hexane and isopropanol at a *n*-hexane:isopropanol ratio of 3:1 (volume ratio).

### [Comparative Example 52]

Procedures were performed in the same manner as in Comparative Example 41, except that the solvent added to the emulsion was 10 mL of a solvent mixture of *n*-hexane and isopropanol at a *n*-hexane:isopropanol ratio of 10:1 (volume ratio).

### [Comparative Example 53]

Procedures were performed in the same manner as in Comparative Example 41, except that the solvent added to the emulsion was 10 mL of a solvent mixture of isopropanol and ethanol at an isopropanol:ethanol ratio of 1:1 (volume ratio).

### [Comparative Example 54]

Procedures were performed in the same manner as in Comparative Example 41, except that the solvent added to the emulsion was 10 mL of a solvent mixture of isopropanol and ethanol at an isopropanol:ethanol ratio of 3:1 (volume ratio).

### [Comparative Example 55]

Procedures were performed in the same manner as in Comparative Example 41, except that the solvent added to the emulsion was 10 mL of a solvent mixture of isopropanol and ethanol at an isopropanol:ethanol ratio of 10:1 (volume ratio).

Table 2 lists the values of the rate of oil collection (%) in Examples 4 to 6 and Comparative Examples 29 to 55. In Examples 4 to 6, the rate of oil collection was 86.2% or more; in particular, in Examples 4 and 5, the rate of oil collection was very high and 91.7% or more. It has been confirmed that the rate of oil collection is 86% or more when the *n-*hexane:ethanol ratio is in the range of 2:1 to 5:1 and 91% or more when the *n*-hexane:ethanol ratio is in the range of 2:1 to 3:1. In Comparative Example 32 where in the step B the yeast was disrupted in the culture fluid, the rate of oil collection was lower than in Reference Example where the yeast was disrupted in purified water, despite the use of the same extraction solvent (*n-*hexane: ethanol = 1:1).

In Comparative Examples 29, 30, and 37, which were different from Examples 4 to 6 only in the solvent used in the step D, the rate of oil collection was 43.9% or less, while in Comparative Examples 31, 35, 36, 38, and 39, the rate of oil collection was 70% or more. In Comparative Examples 35, 38, and 39, the rate of oil collection was more than 80%; in particular, the rate of oil collection in Comparative Example 38 was higher than the rate of oil collection in Example 6.

**[Table 2]**

| Solvent used | With steps C and D | Rate of oil collection (%) | Without steps C and D | Rate of oil collection (%) |
|---|---|---|---|---|
| *n* -Hexane | Comparative Example 29 | 5.5 | Comparative Example 41 | 0 |
| Ethanol | Comparative Example 30 | 43.9 | Comparative Example 42 | 0 |
| Isopropanol | Comparative Example 31 | 71.5 | Comparative Example 43 | 0 |
| *n* -Hexane Ethanol = 1:1 | Comparative Example 32 | 54.9 | Comparative Example 44 | 0 |
| *n* -Hexane Ethanol = 2:1 | Example 4 | 91.7 | Comparative Example 45 | 0 |
| *n* -Hexane Ethanol = 3:1 | Example 5 | **98.4** | Comparative Example 46 | 0 |
| *n* -Hexane Ethanol = 5:1 | Example 6 | **86.2** | Comparative Example 47 | 0 |
| *n* -Hexane Ethanol = 6:1 | Comparative Example 33 | 59.7 | Comparative Example 48 | 0 |
| *n* -Hexane Ethanol = 10:1 | Comparative Example 34 | 56.1 | Comparative Example 49 | 0 |
| *n* -Hexane:Isopropanol= 1:1 | Comparative Example 35 | 82.7 | Comparative Example 50 | 0 |
| *n* -Hexane Isopropanol = 3:1 | Comparative Example 36 | 70.6 | Comparative Example 51 | 0 |
| *n* -Hexane:Isopropanol= 10:1 | Comparative Example 37 | 22.1 | Comparative Example 52 | 0 |
| Isopropanol:Ethanol= 1:1 | Comparative Example 38 | 87.7 | Comparative Example 53 | 0 |
| Isopropanol:Ethanol= 3:1 | Comparative Example 39 | 82.0 | Comparative Example 54 | 0 |
| Isopropanol:Ethanol= 10:1 | Comparative Example 40 | 66.0 | Comparative Example 55 | 0 |

However, in the case where *n*-hexane and isopropanol, or isopropanol and ethanol, were used in the step D, the collected alternative to palm oil had the drawback of being black, having an unusual odor, or containing solids even when heated to 50°C or higher, and lacked quality suitable for replacing palm oil. The drawback was presumably due to incorporation of components of the culture fluid into the alternative to palm oil.

Likewise, in Comparative Example 30 where ethanol was used alone or Comparative Example 31 where isopropanol was used alone, the collected alternative to palm oil had the drawback of being black, having an unusual odor, or containing solids even when heated to 50°C or higher, and lacked quality suitable for use as an edible oil.

### Industrial Applicability

As seen from the foregoing description, the oil production method of the present invention allows for unprecedented efficient collection of an alternative to palm oil from a liquid containing a yeast belonging to the genus *Lipomyces* and is useful as an industrial method for producing an alternative to palm oil that can be used, for example, as an edible oil or a material of cosmetics.

## Claims

1. An oil production method comprising:
a step A of culturing a yeast belonging to the genus *Lipomyces* and allowing the yeast to produce an oil;
a step B of disrupting the yeast belonging to the genus *Lipomyces* and obtaining an emulsion;
a step C of separating the emulsion into a residue and a liquid by passing the emulsion through a non-woven fabric or a woven fabric;
a step D of distributing the oil contained in the residue into n-hexane and ethanol and collecting a n-hexane layer; and
a step E of evaporating the *n*-hexane and collecting the oil, wherein
a volume ratio of the *n*-hexane to the ethanol is from 2:1 to 5:1.

2. The oil production method according to claim 1, wherein the non-woven fabric or the woven fabric has an average pore size of 0.5 to 10 µm and an air permeability of 2 to 45 cm³/(cm²•s).

3. The oil production method according to claim 1, wherein the yeast belonging to the genus *Lipomyces* is *Lipomyces starkeyi.*

4. The oil production method according to any one of claims 1 to 3, wherein in the step C, the emulsion is passed through the non-woven fabric or the woven fabric at a pressure of 5 to 200 kPa.
